# EUROPEAN PATENT APPLICATION

(11) **EP 3 120 854 A1**
(43) Date of publication of application: **25.01.2017**
(21) Application number: 15764466.7
(22) Date of filing: 06.03.2015
(51) Int. Cl.: A61K 31/575, A61K 8/63, A61K 8/97, A61K 36/899, A61P 3/04, A61P 3/06, A61P 3/10, A61P 43/00, A61Q 19/00, C12N 9/99

(54) **CYCLOARTENOL-CONTAINING COMPOSITION**

(30) Priority: 17.03.2014 JP 2014053971; 02.03.2015 JP 2015040186
(71) Applicant: Kao Corporation, Tokyo 103-8210 (JP)
(72) Inventor: HASHIZUME, Kohjiro, Haga-gun Tochigi 321-3497 (JP); OKAHARA, Fumiaki, Haga-gun Tochigi 321-3497 (JP)
(74) Representative: Vossius & Partner Patentanwälte Rechtsanwälte mbB
(86) International application number: PCT/JP2015/056604
(87) International publication number: WO 2015/141493

(57) **Abstract**

Provided is a cycloartenol-containing composition which can be used as a material for food and beverage, cosmetics, or the like, and has high biological activities. The cycloartenol-containing composition has a cycloartenol content of more than 37% by mass, a 24-methylenecycloartanol content of more than 5% by mass, and a sum content of cycloartenol and 24-methylenecycloartanol of more than 76% by mass, relative to the total mass of sterols.

## Description

### Field of the Invention

The present invention relates to a cycloartenol-containing composition usable as a material for food and beverage, pharmaceuticals, cosmetics, or the like.

### Background of the Invention

Steroid is a generic term for compounds having a cyclopentanohydrophenanthrene ring (C₁₇H₂₈), among which a compound having a hydroxyl group at the 3-position and having 27 to 30 carbon atoms is called sterol. Sterols are widely distributed in animals and plants, and they exist as a free form, fatty acid ester form, or glycoside form. As sterols mostly existing in plants, which are so-called plant sterols, β-sitosterol, stigmasterol, and campesterol are known. Sterols having 30 carbon atoms are called triterpene alcohols.

The triterpene alcohols are known as bioactive substances contained in rice bran, and generally a mixture of cycloartenol, 24-methylenecycloartanol, campesterol, cycloartanol, cyclobranol, and the like. Known biological actions of the triterpene alcohols include an improvement in blood cholesterol and blood lipid, enhanced adiponectin secretion, and inhibition of lipid absorption (Patent Literatures 1 to 8).

Cycloartenol is a main component among the rice bran-derived triterpene alcohols. It is reported that a purified product of cycloartenol separated from rice bran extract by silica gel column chromatography exerts inhibitory activities against postprandial blood glucose increase and GIP increase in vivo (Patent Literatures 9 to 11).

### Citation List

[Patent Literature 1] JP-A-57-018617
[Patent Literature 2] JP-A-57-116415
[Patent Literature 3] JP-A-57-027824
[Patent Literature 4] JP-A-61-118318
[Patent Literature 5] JP-A-2001-224309
[Patent Literature 6] JP-A-2005-068132
[Patent Literature 7] JP-A-2004-519228
[Patent Literature 8] JP-A-2006-257064
[Patent Literature 9] JP-A-2012-515139
[Patent Literature 10] JP-A-2012-515140
[Patent Literature 11] JP-A-2012-020941

### Summary of the Invention

The present invention provides a cycloartenol-containing composition having a cycloartenol content of more than 37% by mass, a 24-methylenecycloartanol content of more than 5% by mass, and a sum content of cycloartenol and 24-methylenecycloartanol of more than 76% by mass, relative to the total mass of sterols.

The present invention further provides a lipase inhibitor, a lipid absorption inhibitor, a GIP increase inhibitor, a postprandial blood glucose increase inhibitor, or an obesity inhibitor, which comprises the above-described cycloartenol-containing composition as an active ingredient.

### Detailed Description of the Invention

The above-described chromatographically purified products of cycloartenol are unsuitable for use in food materials. On the other hand, there have been cases where rice bran extract which is a raw material of cycloartenol and is a material usable as food cannot necessarily exert desired biological activities in vivo, even in the case of abundantly comprising cycloartenol.

The present invention relates to providing a cycloartenol-containing composition which can be added not only to pharmaceuticals but also to food and beverage, cosmetics, or the like, as a material, and which has high biological activities.

The inventors prepared cycloartenol-containing compositions having various compositions from rice bran extract and investigated the biological activities thereof in an artificial in vivo system. As a result, they have found that a composition configured so that the content of cycloartenol and the content of 24-methylenecycloartanol respectively fall within specific ranges has high biological activities.

According to the present invention, a cycloartenol-containing composition which can be added not only to pharmaceuticals but also to food and beverage, cosmetics, or the like, as a material, and which can sufficiently exert the biological activities of cycloartenol in vivo can be provided.

The triterpene alcohols which are components of the composition of the present invention are contained in rice (brown rice), rice bran or its extract, common vegetable fats and oils such as soybean oil, corn oil, and rice oil, and plant-derived raw materials such as γ-orizanol. Gamma-orizanol is a mixture of ferulic acid esters of triterpene alcohols extracted from rice oil, and can produce triterpene alcohols by hydrolysis or the like of γ-orizanol.

The cycloartenol-containing composition of the present invention is a composition comprising triterpene alcohols prepared from the above-described plant-derived raw material. The composition of the present invention comprises cycloartenol as a main component and 24-methylenecycloartanol, and may further optionally comprise other triterpene alcohols or plant sterols, such as cycloartanol, stigmasterol, β-sitosterol, and campesterol.

The cycloartenol-containing composition provided by the present invention is desired to be suitable in use as a material for food and beverage. Accordingly, the cycloartenol-containing composition of the present invention is prepared without chromatographic purification using a silica gel column or the like.

Conventional cycloartenol-containing compositions which are prepared from plant-derived raw materials have unstable biological activities in vivo, although they comprise cycloartenol as their main component. Actually, even if conventional cycloartenol-containing compositions have almost the same cycloartenol content ratio, the compositions do not always exert the same activities. For example, it is reported that the purified products of cycloartenol obtained from the above-described plant-derived raw materials by chromatographic purification have inhibitory activities against postprandial blood glucose increase and GIP increase as shown in Patent Literatures 9 to 11. Further, as shown in Examples, which will be described below, purified products of cycloartenol have an action of reducing the amount of free fatty acid produced by lipase acting on the bile acid micelles (Table 3). Meanwhile, commercially available cycloartenol formulations prepared from the same plant-derived raw materials did not necessarily inhibit GIP secretion in vivo or free fatty acid production from the bile acid micelles, and rather increased free fatty acid production in some cases (Table 3).

As a result of various studies on the above-described problems, the inventors have found that compositions having high activity can be obtained by adjusting, in the cycloartenol-containing compositions prepared from the above-described plant-derived raw materials, the cycloartenol content, the sum content of cycloartenol and 24-methylenecycloartanol, and the plant sterol content to specific ranges. Further, these compositions have advantages of being usable suitably as a material to be added to food and beverage, since they can be prepared without undergoing chromatographic purification.

Accordingly, the cycloartenol-containing composition of the present invention has the following composition. Note that % means % by mass.

Relative to the total mass of sterols contained in the composition, the cycloartenol content is more than 37%, the 24-methylenecycloartanol content is more than 5%, and the sum content of cycloartenol and 24-methylenecycloartanol is more than 76%. Suitably, the campesterol content relative to the total mass of sterols contained in the composition is less than 5%. Also suitably, the β-sitosterol content relative to the total mass of sterols contained in the composition is less than 5%. More suitably, both the campesterol content and the β-sitosterol content relative to the total mass of sterols contained in the composition are less than 5%.

Preferably, the cycloartenol-containing composition of the present invention has the following composition. Note that % means % by mass.

Relative to the total mass of sterols contained in the composition, the cycloartenol content is more than 37%, the 24-methylenecycloartanol content is more than 5%, and the sum content of cycloartenol and 24-methylenecycloartanol is more than 76% and less than 96%. Suitably, the campesterol content relative to the total mass of sterols contained in the composition is less than 5%. Also suitably, the β-sitosterol content relative to the total mass of sterols contained in the composition is less than 5%. More suitably, both the campesterol content and the β-sitosterol content relative to the total mass of sterols contained in the composition are less than 5%.

Further preferably, the cycloartenol-containing composition of the present invention has the following composition. Note that % means % by mass.

Relative to the total mass of sterols contained in the composition, the cycloartenol content is more than 42%, the 24-methylenecycloartanol content is more than 5%, and the sum content of cycloartenol and 24-methylenecycloartanol is more than 81% and less than 96%. Suitably, the campesterol content relative to the total mass of sterols contained in the composition is less than 5%. Also suitably, the β-sitosterol content relative to the total mass of sterols contained in the composition is less than 5%. More suitably, both the campesterol content and the β-sitosterol content relative to the total mass of sterols contained in the composition are less than 5%.

In this description, the content of the triterpene alcohols or plant sterols contained in the composition mean the content thereof in their free form, unless otherwise specified. For example, the cycloartenol content in the composition means the content of cycloartenol existing in its free form in the composition. In the cycloartenol-containing composition of the present invention, the above-described triterpene alcohols or plant sterols not only in the free form but also in the form of derivatives, esters such as fatty acid ester, ferulic acid ester, and cinnamic acid ester, and glycosides such as saponin may be contained in some cases, but the content of these derivatives does not include the content of the triterpene alcohols or plant sterols in the composition of the present invention. The content of free triterpene alcohols or free plant sterols in the composition can be measured by gas chromatography.

The above-described cycloartenol-containing composition of the present invention is preferably a composition prepared without chromatographic purification. In this description, the chromatographic purification means a purification method in which a crude composition which has once been adsorbed to an adsorption carrier such as silica gel or ODS silica gel is subjected to elution development with a solvent or the like, thereby achieving separation and purification by the difference in adsorbability on the adsorption carrier. Examples thereof include silica gel column chromatography, ODS column chromatography, high-performance liquid chromatography (HPLC), and thin-layer chromatography (TLC). Accordingly, the cycloartenol-containing composition of the present invention is substantially free from components which can be incorporated due to chromatographic purification, such as chloroform, ethyl acetate, methanol, and acetonitrile, which are a developing solvent for chromatography, and therefore is a safe composition when being added to food.

The cycloartenol-containing composition of the present invention can be a composition prepared from any one of rice (brown rice), rice bran or its extract, general vegetable fat or oils such as soybean oil, corn oil, and rice oil, and plant-derived raw materials such as γ-orizanol, or mixtures thereof.

The class or variety of rice which can be a raw material of the triterpene alcohols is not specifically limited, and any one of glutinous rice, non-glutinous rice, red rice, black and purple rice and the like, for example, from a japonica, indica, or javanica variety can be used. The brown rice is composed of parts including the seed coat, pericarp, endosperm, and germ of gramineous rice and has an aleurone layer as the outer layer of the endosperm tissue. Examples of the rice bran include any one of parts other than endosperm which are removed when brown rice is pearled (that is, seed coat, pericarp, starch layer, and germ), or mixtures thereof.

In this description, the rice bran extract includes rice oil, unless otherwise specified. The rice oil is a vegetable oil extracted from rice bran serving as a raw material. As an extraction process of the rice bran extract, a solvent extraction process performed by impregnation in a solvent at room temperature or under heating, or using an extractor such as a Soxhlet extractor, an extraction process using a distillation process such as steam distillation, a supercritical extraction process performed by bringing carbon dioxide gas into the supercritical state, a compression process in which an extract is obtained by pressing, or the like, can be used.

Examples of the extraction solvent used for the solvent extraction of the rice bran include ethanol, hexane, pentane, supercritical carbon dioxide, vegetable fat or oil, animal fat or oil, algae oil, and squalene. These can be used individually or in combination of two or more, and it is also possible to repeat the process while changing the solvent. Among these, a fat-soluble solvent such as hydrocarbons is preferably used, and hexane is particularly preferably used.

Examples of the extraction process of vegetable fat or oil such as soybean oil, corn oil, and rice oil include compression or solvent extraction. For example, oil can be produced by extracting oil by adding a fat-soluble solvent such as hexane to a ground product of a plant raw material such as rice bran, removing the solid content by filtration, and thereafter removing the solvent by distillation.

Preferably, the above-described rice bran extract or vegetable fat or oil is further subjected to alcohol extraction. Examples of the alcohol for alcohol extraction include methanol and ethanol, but ethanol is preferable.

Gamma-orizanol can be produced, for example, by extracting rice oil with an alcohol such as methanol or ethanol. Alternatively, commercially available purified products or crudely purified products of γ-orizanol (such as products manufactured by ORYZA OIL & FAT CHEMICAL CO., LTD.) can be used as γ-orizanol.

Preferably, the above-described rice bran extract, the above-described vegetable fat or oil, the alcohol extract thereof, or γ-orizanol is further hydrolyzed. The hydrolysis causes free triterpene alcohols to be produced. The hydrolysis treatment can be performed using acid, alkali, or enzyme, and among these, the hydrolysis using alkali is preferable.

Examples of the hydrolysis treatment using acid include a method in which the treatment solution is adjusted to have a pH of generally from 0 to 1 using hydrochloric acid, sulfuric acid, or the like, and is allowed to react at from 25 to 200°C for from 0.5 to 50 hours. Examples of the hydrolysis treatment using alkali include a method in which the treatment solution is adjusted to have a pH of generally from 12 to 14 using sodium hydroxide, potassium hydroxide, calcium hydroxide, sodium carbonate, or the like, and is allowed to react at from 25 to 200°C for from 0.5 to 50 hours. Examples of the enzyme used for the enzymatic hydrolysis include lipase, cholesterol esterase, and oryzanol esterase. Examples of the hydrolysis treatment using enzyme include a method in which an appropriate amount of the above-described enzyme is allowed to react with a substrate for from 0.5 to 50 hours under conditions near the optimum pH and the optimum temperature of the enzyme.

Alternatively, examples of the method for producing the hydrolysate of rice bran include methods disclosed in JP-B-55-002440, JP-A-2006-273764, and JP-A-2006-257064. Among these, a method disclosed in JP-A-2006-257064 in which the rice bran is dissolved in ethanol by heating (at 80°C for 1 hour), followed by concentration, hexane is thereafter added and dissolved therein, sulfuric acid is further added thereto to give a pH of 3, followed by filtration, the filtrate is subjected to reflux extraction using ethanol and sodium hydroxide (at 80°C for 1 hour), and hydrochloric acid is added to the supernatant which has been allowed to stand still to be neutralized and concentrated to dryness is suitable. Alternatively, commercially available products (such as "Oryza triterpenoid-P", ORYZA OIL & FAT CHEMICAL CO., LTD.) can be used as the hydrolysate of rice bran.

The above-described hydrolysate contains triterpene alcohols: cycloartane-type triterpenes such as cycloartenol, 24-methylenecycloartanol, cyclobranol, cycloartanol, and 9,19-cyclolanostane-23,25-dien-3-ol; lupane-type triterpenes; cucurbitane-type triterpenes; and plant sterols such as β-sitosterol and campesterol.

Preferably, the cycloartenol-containing composition of the present invention can be rice bran extract, alcohol extract of vegetable fat or oil (such as soybean oil, corn oil, and rice oil), or a mixture of the alcohol extract. Examples of the alcohol for alcohol extraction include methanol and ethanol, but ethanol is preferable. More preferably, the cycloartenol-containing composition of the present invention can be a hydrolysate of alcohol extract of rice bran extract or vegetable fat or oil (such as soybean oil, corn oil, and rice oil), or a mixture of the hydrolysate. Examples of the alcohol for alcohol extraction include methanol and ethanol, but ethanol is preferable. As the hydrolysate, an alkaline hydrolysate is preferable. The alcohol extract or the hydrolysate thereof may be purified by recrystallization.

Further preferably, the cycloartenol-containing composition of the present invention is alcohol extract of rice bran extract, or a hydrolysate of the alcohol extract, and is a composition prepared without chromatographic purification. Accordingly, the cycloartenol-containing composition of the present invention can comprise a slight amount of components which are contained in rice bran extract but are removed from a chromatographically purified product. Examples of the slight amount of components include cycloeucalenol, stigmasterol, gramisterol, citrostadienol, parkeol, methyl-ergosta-8,14,24(28)-trien-3-ol, 9,19-cyclolanosta-23,25-dien-3-ol, 9,19-cyclolanosta-25-ene-3,24-diol, 9,19-cyclolanosta-23-ene-3,25-diol, and 24-methylene-9,19-cyclolanostane-3,28-diol. The cycloartenol-containing composition of the present invention can comprise the above-listed slight amount of components in a total amount of from 0.1 to 0.5% by mass.

The form of the cycloartenol-containing composition of the present invention is not specifically limited, and may be a fluid in liquid form or paste form, or a solid in gel form or powder form, or may be in the form in which the fluid or solid is enclosed in a vesicle such as liposome or a microcapsule.

The cycloartenol-containing composition of the present invention having the above-described specific composition has an action of significantly reducing the amount of free fatty acid produced by lipase acting on the bile acid micelles, is effective in inhibiting lipase in vivo and inhibiting lipid absorption into cells, and is further useful in inhibiting GIP secretion elicited by lipid absorption (or inhibiting blood GIP concentration increase). Further, the cycloartenol-containing composition of the present invention can have effects such as inhibition of postprandial blood glucose increase and obesity inhibition as a result of the lipid absorption inhibition and the GIP secretion inhibition, and is therefore useful for preventing, ameliorating, or treating insulin resistance, adiposity, arteriosclerosis, diabetes, hyperlipidemia, or the like.

Accordingly, the cycloartenol-containing composition of the present invention can be used as an active ingredient for lipase inhibition, lipid absorption inhibition, GIP secretion inhibition, inhibition of postprandial blood glucose increase, or obesity inhibition. Alternatively, the cycloartenol-containing composition of the present invention can be used also as an active ingredient for preventing, ameliorating, or treating diseases such as insulin resistance, adiposity, arteriosclerosis, diabetes, and hyperlipidemia. Subjects directed to the prevention, amelioration, or treatment of diabetes include patients (or affected animals) with prediabetes and patients (or affected animals) with diabetes. A preventive effect on diabetes can be expected for the former, and an ameliorative effect on diabetes can be expected for the latter.

Subjects to which the cycloartenol-containing composition of the present invention can be applied include humans and nonhuman animals. Examples of the nonhuman animals preferably include non-human mammals and birds, such as dogs, cats, hamsters, mice, rats, guinea pigs, horses, cows, pigs, goats, sheep, monkeys, chickens, ducks, dabbling ducks, geese, and turkeys.

The cycloartenol-containing composition of the present invention may be therapeutically used, or may be non-therapeutically used. In this description, the term "non-therapeutically" means a concept which does not include medical practices, that is, does not include a process in which an operation, treatment or diagnosis is performed on human, more specifically, does not include a process in which an operation, treatment or diagnosis is performed on human by a doctor, a health professional, or those who are instructed by a doctor. Examples of the non-therapeutic use include use of the cycloartenol-containing composition of the present invention in healthy human or animals for lipase inhibition, lipid absorption inhibition, GIP secretion inhibition, inhibition of postprandial blood glucose increase, or obesity inhibition, for the purpose of maintaining or enhancing health, or for aesthetic purpose.

Further, the cycloartenol-containing composition of the present invention can be used for producing pharmaceutical products, quasi drugs, cosmetics, food and beverage, feed, or the like, for the purpose of lipase inhibition, lipid absorption inhibition, GIP secretion inhibition, inhibition of postprandial blood glucose increase, or obesity inhibition, or for the purpose of preventing, ameliorating, or treating diseases such as insulin resistance, adiposity, arteriosclerosis, diabetes, and hyperlipidemia. The pharmaceutical products, quasi drugs, cosmetics, food and beverage, feed, or the like, can be produced or used for human or nonhuman animals. The types of nonhuman animals are as described above.

The above-described pharmaceutical products, quasi drugs, and cosmetics comprise the cycloartenol-containing composition of the present invention as an active ingredient for obtaining effects such as lipase inhibition, lipid absorption inhibition, GIP secretion inhibition, inhibition of postprandial blood glucose increase, and obesity inhibition, or effects of preventing, ameliorating, or treating diseases such as insulin resistance, adiposity, arteriosclerosis, diabetes, and hyperlipidemia. The pharmaceutical products, quasi drugs, and cosmetics may further comprise other active ingredients, pharmaceutical ingredients, cosmetic ingredients, or the like, as long as the above-described action on the biological activities by the composition of the present invention is not lost. The pharmaceutical products, quasi drugs, and cosmetics can be produced by a conventional method from the composition of the present invention, or by combining carriers which are pharmaceutically or cosmetically acceptable, the above-described other active ingredients, pharmaceutical ingredients, and cosmetic ingredients, and the like, as needed. The pharmaceutical products, quasi drugs, and cosmetics can be prepared in any dosage form for oral administration or parenteral administration, but are preferably prepared in the form of oral administration. Examples of the dosage form of oral administration include tablets, capsules, granules, powders, and syrups, and examples of the dosage form of parenteral administration include injections, suppositories, inhaled drugs, transdermal drugs, and external medicine.

The above-described food and beverage, and feed are intended to obtain functions such as lipase inhibition, lipid absorption inhibition, GIP secretion inhibition, inhibition of postprandial blood glucose increase, obesity inhibition, or the like, and can be food and beverage, functional food and beverage, supplement food, food and beverage for the sick, food and beverage for specified health use, feed, pet food, or the like, which are labeled with such a function, as needed. It should be noted that the food and beverage, functional food and beverage, supplement food, food and beverage for the sick, and food and beverage for specified health use, which are labeled with such a function, as needed, refer to food and beverage which are permitted to display their functions and are distinguished from general food and beverage. The above-described food and beverage, and feed comprise the cycloartenol-containing composition of the present invention as an active ingredient for obtaining actions such as lipase inhibition, lipid absorption inhibition, GIP secretion inhibition, inhibition of postprandial blood glucose increase, or obesity inhibition. The above-described food and beverage, and feed include any form and type of food, beverage, and feed.

The content of the cycloartenol-containing composition of the present invention in the above-described pharmaceutical products, quasi drugs, cosmetics, food and beverage, or feed is preferably 0.01 to 100% by mass, more preferably from 0.1 to 100% by mass, further preferably from 1 to 100% by mass, further preferably from 10 to 100% by mass, in terms of the cycloartenol content.

The dose or intake of the cycloartenol-containing composition of the present invention may be an effective amount in which effects such as lipase inhibition, lipid absorption inhibition, GIP secretion inhibition, inhibition of postprandial blood glucose increase, and obesity inhibition, or effects of preventing, ameliorating, or treating diseases such as insulin resistance, adiposity, arteriosclerosis, diabetes, and hyperlipidemia are obtained. The dose or intake of the cycloartenol-containing composition of the present invention can vary depending on the state, body weight, gender, and age of the subject, or other factors, but is preferably from 1 µg to 100 mg/kg/day, more preferably from 10 µg to 100 mg/kg/day, further preferably from 50 µg to 100 mg/kg/day, still further preferably from 50 µg to 10 mg/kg/day, in terms of the cycloartenol content, in the case of oral administration in an adult. Further, the above-described formulation can be administered according to any dosing regimen, but is preferably administered once to several times a day. In the case of administration or intake for the purpose of inhibition of postprandial blood glucose increase, administration or intake before or between food intakes or feed intakes is preferable, and administration or intake within 5 minutes to 30 minutes before food intakes or feed intake is more preferable.

As exemplary embodiments of the present invention, the following compositions, uses, or methods are further shown in this description.
[1] A cycloartenol-containing composition having a cycloartenol content of more than 37% by mass, a 24-methylenecycloartanol content of more than 5% by mass, and a sum content of cycloartenol and 24-methylenecycloartanol of more than 76% by mass, relative to the total mass of sterols.
[2] The cycloartenol-containing composition according to [1], preferably having a sum content of cycloartenol and 24-methylenecycloartanol of less than 96% by mass.
[3] The cycloartenol-containing composition according to [1] or [2], preferably having a cycloartenol content of more than 42% by mass, and a sum content of cycloartenol and 24-methylenecycloartanol of more than 81% by mass and less than 96% by mass, relative to the total mass of sterols.
[4] The cycloartenol-containing composition according to any one of [1] to [3], preferably having a campesterol content or a sitosterol content of less than 5 mass%, relative to the total mass of sterols.
[5] The cycloartenol-containing composition according to [4], preferably having both a campesterol content and a sitosterol content of less than 5% by mass, relative to the total mass of sterols.
[6] The cycloartenol-containing composition according to any one of [1] to [5], the composition preferably being a hydrolysate of alcohol extract of rice bran extract or vegetable fat and oil, or a mixture thereof.
[7] The cycloartenol-containing composition according to any one of [1] to [6], the composition preferably being the hydrolysate of the alcohol extract of the rice bran extract.
[8] The cycloartenol-containing composition according to any one of [1] to [7], the composition preferably being a composition prepared from the rice bran extract without chromatographic purification.
[9] A lipase inhibitor comprising the cycloartenol-containing composition according to any one of [1] to [8] above as an active ingredient.
[10] A lipid absorption inhibitor comprising the cycloartenol-containing composition according to any one of [1] to [8] above as an active ingredient.
[11] A GIP secretion inhibitor comprising the cycloartenol-containing composition according to any one of [1] to [8] above as an active ingredient.
[12] An inhibitor of postprandial blood glucose increase comprising the cycloartenol-containing composition according to any one of [1] to [8] above as an active ingredient.
[13] An obesity inhibitor comprising the cycloartenol-containing composition according to any one of [1] to [8] above as an active ingredient.
[14] An agent for preventing, ameliorating, or treating a disease selected from the group consisting of insulin resistance, adiposity, arteriosclerosis, diabetes, and hyperlipidemia, the agent comprising the cycloartenol-containing composition according to any one of [1] to [8] above as an active ingredient.
[15] Use of the cycloartenol-containing composition according to any one of [1] to [8] above for producing a lipase inhibitor.
[16] Use of the cycloartenol-containing composition according to any one of [1] to [8] above for producing a lipid absorption inhibitor.
[17] Use of the cycloartenol-containing composition according to any one of [1] to [8] above for producing a GIP secretion inhibitor.
[18] Use of the cycloartenol-containing composition according to any one of [1] to [8] above for producing an inhibitor of postprandial blood glucose increase.
[19] Use of the cycloartenol-containing composition according to any one of [1] to [8] above for producing an obesity inhibitor.
[20] Use of the cycloartenol-containing composition according to any one of [1] to [8] above for producing an agent for preventing, ameliorating, or treating a disease selected from the group consisting of insulin resistance, adiposity, arteriosclerosis, diabetes, and hyperlipidemia.
[21] Use of the cycloartenol-containing composition according to any one of [1] to [8] above for lipase inhibition.
[22] Use of the cycloartenol-containing composition according to any one of [1] to [8] above for lipid absorption inhibition.
[23] Use of the cycloartenol-containing composition according to any one of [1] to [8] above for GIP secretion inhibition.
[24] Use of the cycloartenol-containing composition according to any one of [1] to [8] above for inhibition of postprandial blood glucose increase.
[25] Use of the cycloartenol-containing composition according to any one of [1] to [8] above for obesity inhibition.
[26] The use according to any one of [21] to [25], the use preferably being non-therapeutic use.
[27] The cycloartenol-containing composition according to any one of [1] to [8] above for use in lipase inhibition.
[28] The cycloartenol-containing composition according to any one of [1] to [8] above for use in lipid absorption inhibition.
[29] The cycloartenol-containing composition according to any one of [1] to [8] above for use in GIP secretion inhibition.
[30] The cycloartenol-containing composition according to any one of [1] to [8] above for use in inhibition of postprandial blood glucose increase.
[31] The cycloartenol-containing composition according to any one of [1] to [8] above for use in obesity inhibition.
[32] The cycloartenol-containing composition according to any one of [1] to [8] above for use in preventing, ameliorating, or treating a disease selected from the group consisting of insulin resistance, adiposity, arteriosclerosis, diabetes, and hyperlipidemia.
[33] A method for inhibiting lipase, comprising administering the cycloartenol-containing composition according to any one of [1] to [8] above to a subject in an effective amount.
[34] A method for inhibiting lipid absorption, comprising administering the cycloartenol-containing composition according to any one of [1] to [8] above to a subject in an effective amount.
[35] A method for inhibiting GIP secretion, comprising administering the cycloartenol-containing composition according to any one of [1] to [8] above to a subject in an effective amount.
[36] A method for inhibiting postprandial blood glucose increase, comprising administering the cycloartenol-containing composition according to any one of [1] to [8] above to a subject in an effective amount.
[37] A method for inhibiting obesity, comprising administering the cycloartenol-containing composition according to any one of [1] to [8] above to a subject in an effective amount.
[38] A method for preventing, ameliorating, or treating a disease selected from the group consisting of insulin resistance, adiposity, arteriosclerosis, diabetes, and hyperlipidemia, the method comprising administering the cycloartenol-containing composition according to any one of [1] to [8] above to a subject in an effective amount.
[39] The method according to any one of [33] to [37], the method preferably being a non-therapeutic method.

### [Examples]

Hereinafter, the present invention will be further specifically described by way of Examples. In Examples below, the following abbreviations may be used.
CA: Cycloartenol
24Me: 24-methylene-cycloartanol
CS: Campesterol
β-SS: β-sitosterol

### Reference Example 1: Preparation of purified product

As the purified products of cycloartenol and 24-methylene-cycloartanol, products obtained by purifying a commercially available triterpene alcohol formulation (product name: Oryza triterpenoid P, obtained from ORYZA OIL & FAT CHEMICAL CO., LTD.) as a raw material were used. Specifically, 5 g of the triterpene alcohol formulation was fractionated by silica gel column chromatography (developing solvent: hexane/ethyl acetate = 9/1) to obtain 3.93 g of a fraction comprising cycloartenol and 24-methylene-cycloartanol. Thereafter, 1.4 g of the fraction was fractionated by HPLC (developing solvent: methanol/acetonitrile/tetrahydrofuran/water = 15/2/2/1) using an ODS column (Inertsil ODS-3: GL Sciences Inc.) to obtain 480 mg of a purified product of cycloartenol and 764 mg of a purified product of 24-methylene-cycloartanol. The purified products respectively had a purity of CA = 99.7% and 24Me = 100.0%.

### Reference Example 2: Quantitative determination of sterols in test samples

About 25 mg of a sample was accurately taken into a 10-mL volumetric flask and was diluted with CHCl₃. 2 µL of the resultant solution was injected into a gas chromatography analyzer (Agilent 6890N network GC system), and analysis was performed under the following conditions. The components were determined by the absolute quantitative determination. The amount of 24-methylene-cycloartanol was calculated from a standard curve plotted using the purified product of 24-methylene-cycloartanol described in Reference Example 1. The amounts of other sterol components (cycloartenol, campesterol, and β-sitosterol) were calculated from standard curves plotted using the purified product of cycloartenol described in Reference Example 1.
Conditions for gas chromatographic analysis
Column: Capillary GC column DB-1 (J&W), 30 m x 0.25 mm, Film thickness: 0.25 µm (which was used after aging overnight at 320°C)
Carrier gas: Helium, 2.30 mL/minute (Average linear speed = 50 cm/second)
Injector: Split ratio: 40:1, T = 300°C, P = 30.28 psi, Total flow = 96.9 mL/minute
Detector: FID, T = 300°C, H₂ flow = 40.0 mL/minute, Air flow = 400.0 mL/minute
Oven temperature: Kept at 150°C for 1.5 minutes, increased to 250°C at 15°C /minute, then increased to 320°C at 5°C /minute, kept for 20 minutes

### Reference Example 3: Quantitative determination of sterols in commercially available triterpene alcohol formulation

Commercially available triterpene alcohol formulations (commercially available products 1 to 3, product name: Oryza triterpenoid P, obtained from ORYZA OIL & FAT CHEMICAL CO., LTD.) were prepared. None of these formulations are products processed by chromatography. The amounts of sterols in the formulations were determined by the procedure according to Reference Example 2. The sterol composition of the formulations is shown in Table 1. It should be noted that the total mass is adjusted in Table 1 so that the amount of CA is 30 mg.

### Preparation Example 1

50 mL of a 99.5% ethanol was added to 5 g of the commercially available product 3, which was heated at 70 to 75°C under stirring to be dissolved. After the dissolution, the obtained composition was air-cooled to room temperature, followed by cooling to 5°C (15 hours) to allow recrystallization. The precipitated crystal was collected by suction filtration, followed by drying under reduced pressure (white powder, 2.33 g). The amounts of sterols in the obtained composition were determined by the procedure according to Reference Example 2. The sterol composition in the composition is shown in Table 1. It should be noted that the total mass is adjusted in Table 1 so that the amount of CA is 30 mg.

### Preparation Example 2

15 mL of a 99.5% ethanol was added to 1.5 g of the composition obtained in Preparation Example 1, which was heated at 70 to 75°C under stirring to be dissolved. After the dissolution, the obtained composition was air-cooled to room temperature, followed by cooling to 5°C (15 hours) to allow recrystallization. The precipitated crystal was collected by suction filtration, followed by drying under reduced pressure (white powder, 0.67 g). The amounts of sterols in the obtained composition were determined by the procedure according to Reference Example 2. The sterol composition in the composition is shown in Table 1. It should be noted that the total mass is adjusted in Table 1 so that the amount of CA is 30 mg.

### Compositions A to J

10 types of compositions were prepared from commercially available triterpene alcohol formulations to obtain compositions of Comparative Examples (compositions A to F) and compositions of the present invention (compositions G to J). The sterol composition of each composition is shown in Table 1. It should be noted that the total mass is adjusted in Table 1 so that the amount of CA is 30 mg.

**[Table 1]**

| Sterol composition of test samples | | | | | | | | | | | |
|---|---|---|---|---|---|---|---|---|---|---|---|
| Sample | | CA | | 24Me | | CA+ 24Me | CS | | β-SS | | Total mass (mg) |
| | | mg | % | mg | % | % | mg | % | mg | % | |
| 1 | Control (olive oil alone) | - | - | - | - | - | - | - | - | - | - |
| 2 | Purified product of cycloartenol *¹ | 30 | 100 | - | 0 | 100 | - | 0 | - | 0 | 30 |
| 3 | Commercially available product 1 *² | 30 | 20 | 51 | 34 | 54 | 23 | 15 | 18 | 12 | 150 |
| 4 | Commercially available product 2 *² | 30 | 33 | 39 | 43 | 76 | 10 | 11 | 3 | 3 | 91 |
| 5 | Commercially available product 3 *² | 30 | 34 | 38 | 43 | 77 | 9 | 10 | 2 | 2 | 88 |
| 6 | Preparation Example 1 | 30 | 38 | 42 | 53 | 91 | 3 | 4 | 2 | 2 | 79 |
| 7 | Preparation Example 2 | 30 | 37 | 47 | 58 | 95 | 1 | 1 | 1 | 1 | 81 |
| 8 | Composition A (Comparative Example) | 30 | 25 | 51 | 42 | 67 | 23 | 19 | 18 | 15 | 122 |
| 9 | Composition B (Comparative Example) | 30 | 31 | 51 | 52 | 82 | - | 0 | 18 | 18 | 99 |
| 10 | Composition C (Comparative Example) | 30 | 32 | 51 | 54 | 86 | - | 0 | 14 | 15 | 95 |
| 11 | Composition D (Comparative Example) | 30 | 39 | 26 | 34 | 72 | 12 | 16 | 9 | 12 | 77 |
| 12 | Composition E (Comparative Example) | 30 | 42 | - | 0 | 42 | 23 | 32 | 18 | 25 | 71 |
| 13 | Composition F (Comparative Example) | - | 0 | 51 | 55 | 55 | 23 | 25 | 18 | 20 | 92 |
| 14 | Composition G | 30 | 37 | 51 | 63 | 100 | - | 0 | - | 0 | 81 |
| 15 | Composition H | 30 | 42 | 26 | 35 | 76 | - | 0 | 18 | 24 | 74 |
| 16 | Composition I | 30 | 47 | 26 | 40 | 86 | - | 0 | 9 | 14 | 65 |
| 17 | Composition J | 30 | 37 | 39 | 48 | 85 | 10 | 12 | 3 | 4 | 82 |

| | | | | | | | | | | | |
|---|---|---|---|---|---|---|---|---|---|---|---|
| *1: Reference Example 1 *2: Purchased from ORYZA OIL & FAT CHEMICAL CO., LTD | | | | | | | | | | | |

### Example 1: Inhibitory action against lipase

Intake of fats is followed by emulsification due to the action of bile acid in the digestive tract to form bile acid micelles. When lipase acts on the micelles, fats are degraded into fatty acid and acyl glycerol to be absorbed by the small intestine cells. In this example, an emulsification liquid containing bile acid micelles was produced as an artificial in vivo system. In the artificial in vivo system, the inhibitory action of the test samples against the lipolytic activity by lipase in vivo was evaluated by measuring the amount of free fatty acid collected from the bile acid micelles after application of lipase in the presence of the test samples.

### (Measurement of amount of free fatty acid produced)

As the test samples, a purified product of cycloartenol, a commercially available product 1, Preparation Example 1, and Preparation Example 2 were used. 10 g of olive oil (Wako Pure Chemical Industries, Ltd.) was added to each test sample (30 mg in terms of CA amount), which was heated at 70°C in an oven for 20 minutes to be dissolved. As the bile acid solution, a solution obtained by taking 48.3 mg of sodium deoxycholate (Wako Pure Chemical Industries, Ltd.), 513 mg of sodium chloride (Wako Pure Chemical Industries, Ltd.), 30.6 mg of calcium chloride dihydrate (KANTO CHEMICAL CO., INC.), and 50 mL of 1N TRIS-HCl buffer (pH = 8) (Wako Pure Chemical Industries, Ltd.), and diluting them with ion-exchange water to give a total amount of 960 g was used.

0.92 g of olive oil in which each test sample was dissolved, 40 mg of egg yolk lecithin (Wako Pure Chemical Industries, Ltd.), and 24 mL of the bile acid solution were added to a 50-mL tube, followed by cooling with ice, and thereafter emulsification was performed thereon using an ultrasonic homogenizer. Then, after cooling with ice for 12.5 minutes, it was pre-incubated at room temperature for 47.5 minutes. Thereafter, 2.5 mL of a 0.1% lipase solution (0.1N TRIS-HCl buffer solution of lipase from porcine pancreas [SIGMA]) was added thereto, which was mixed with stirring using a vortex mixer for 10 seconds, followed by incubation in a water bath at 37°C for 10 minutes. The tube was moved into an oil bath at 70°C, was subjected to enzyme inactivation for 15 minutes, and thereafter was mixed again with stirring using a vortex mixer to collect 100 µL. This was diluted with 99.5% EtOH to 10 times, from which 100 µL was collected again.

The amount of free fatty acid in the reaction solution was determined using NEFA-C Test wako. That is, 800 µL of a coloring reagent A was added to 100 µL of the sample, which was stirred using a vortex mixer, and thereafter was heated at 37°C for 10 minutes. After cooling, 1600 µL of a coloring reagent B was added thereto, followed by stirring, and was heated again at 37°C for 10 minutes. This was subjected to an ultraviolet/visible spectrophotometer to measure an absorbance at 550 nm. Thus, the amount of free fatty acid produced was obtained. Each reaction solution was measured 3 times or 4 times by the same operation.

The results are shown in Table 2. In the samples in which both the content ratio of CA and the content ratio of (CA+24Me) were high, and the content ratio of plant sterols such as CS and β-SS was low (Preparation Examples 1 and 2; and Sample Nos. 6 and 7), the amount of free fatty acid produced in the reaction solution was reduced as in the purified product of cycloartenol (Sample No. 2), and lipase inhibitory activity was confirmed. On the other hand, in the commercially available product 1 in which the CA content ratio was low (Sample No. 3), the amount of free fatty acid produced increased compared to the control.

**[Table 2]**

| Sample | | Sterol composition (% relative to the total mass of sterols) | | | | | Amount of free fatty acid produced | |
|---|---|---|---|---|---|---|---|---|
| | | CA | 24Me | CA+24Me | CS | β-SS | Average | Standard error |
| 1 | Control (olive oil alone) | - | - | - | - | - | 0.376 | 0.007 |
| 2 | Purified product of cycloartenol | 100 | 0 | 100 | 0 | 0 | 0.276 | 0.031 |
| 3 | Commercially available product 1 | 20 | 34 | 54 | 15 | 12 | 0.388 | 0.020 |
| 6 | Preparation Example 1 | 38 | 53 | 91 | 4 | 2 | 0.296 | 0.021 |
| 7 | Preparation Example 2 | 37 | 58 | 95 | 1 | 1 | 0.309 | 0.020 |

### Example 2: Inhibitory action against lipase

In the same manner as in Example 1, the lipase inhibitory activity of various test samples was evaluated based on the amount of free fatty acid produced. As the test samples, the compositions shown in Table 3 were used. The results are shown in Table 3. In the samples in which both the content ratio of CA and the content ratio of (CA+24Me) were high and the content ratio of plant sterols such as CS and β-SS was low (Sample Nos. 14 to 17), the amount of free fatty acid produced in the reaction solution was reduced as in the purified product of cycloartenol (Sample No. 2), and lipase inhibitory activity was confirmed. On the other hand, in the case where the CA content ratio was low, or in the case where the content ratio of (CA+24Me) was low or the content ratio of plant sterols was high, even though the CA content ratio was high (Sample Nos. 3 to 5 and 8 to 13), the amount of free fatty acid produced was the same or rather tended to increase, as compared with the control.

**[Table 3]**

| Sample | | Sterol composition (% relative to the total mass of sterols) | | | | | Amount of free fatty acid produced | |
|---|---|---|---|---|---|---|---|---|
| | | CA | 24Me | CA+24Me | CS | β-SS | Average | Standard error |
| 1 | Control (olive oil alone) | - | - | - | - | - | 0.296 | 0.025 |
| 2 | Purified product of cycloartenol | 100 | 0 | 100 | 0 | 0 | 0.238 | 0.036 |
| 3 | Commercially available product 1 | 20 | 34 | 54 | 15 | 12 | 0.352 | 0.023 |
| 4 | Commercially available product 2 | 33 | 43 | 76 | 11 | 3 | 0.314 | 0.026 |
| 5 | Commercially available product 3 | 34 | 43 | 77 | 10 | 2 | 0.305 | 0.027 |
| 8 | Composition A (Comparative Example) | 25 | 42 | 67 | 19 | 15 | 0.326 | 0.023 |
| 9 | Composition B (Comparative Example) | 31 | 52 | 82 | 0 | 18 | 0.315 | 0.035 |
| 10 | Composition C (Comparative Example) | 32 | 54 | 86 | 0 | 15 | 0.295 | 0.012 |
| 11 | Composition D (Comparative Example) | 39 | 34 | 72 | 16 | 12 | 0.303 | 0.027 |
| 12 | Composition E (Comparative Example) | 42 | 0 | 42 | 32 | 25 | 0.315 | 0.034 |
| 13 | Composition F (Comparative Example) | 0 | 55 | 55 | 25 | 20 | 0.321 | 0.021 |
| 14 | Composition G | 37 | 63 | 100 | 0 | 0 | 0.263 | 0.045 |
| 15 | Composition H | 42 | 35 | 76 | 0 | 24 | 0.266 | 0.015 |
| 16 | Composition I | 47 | 40 | 86 | 0 | 14 | 0.254 | 0.015 |
| 17 | Composition J | 37 | 48 | 85 | 12 | 4 | 0.271 | 0.025 |

### Example 3: Inhibitory action against blood GIP increase

### (Animals)

For experiments, C57BL/6J mice (male, 8 week-old, manufactured by CLEA Japan, Inc.) grouped so that each group has the same body weight (N = 16/group) were used and were subjected to the tests.

### (Samples)

Starch (waxy corn, Amioca) was dissolved in distilled water in an amount 4 times
the amount of the starch and gelatinized (pregelatinized) by autoclaving (at 121°C under a pressure of 2 atmospheres for 20 minutes). Each of the test samples (Sample Nos. 2 to 4 and 8) shown in Table 4 was emulsified in triolein containing egg yolk lecithin and albumin to obtain an emulsified oil. The above-described α-starch and emulsified oil were mixed and was uniformly ground using a homogenizer to prepare a starch paste sample containing 10% of carbohydrate, 5% of lipid, and 175 µM of the test sample in terms of CA (Table 5).

**[Table 4]**

| Sterol composition of test samples (%) | | | | | | |
|---|---|---|---|---|---|---|
| | Sample | CA | 24Me | β-SS | CS | Others |
| 2 | Purified product of cycloartenol *¹ | 100 | 0 | 0 | 0 | 0 |
| 3 | Commercially available product 1 *² | 20 | 34 | 12 | 15 | 19 |
| 4 | Commercially available product 2 *² | 33 | 43 | 3 | 11 | 10 |
| 8 | Composition A (Comparative Example) | 25 | 42 | 15 | 19 | 0 |

| | | | | | | |
|---|---|---|---|---|---|---|
| *1: Reference Example 1 *2: Purchased from ORYZA OIL & FAT CHEMICAL CO., LTD. | | | | | | |

**[Table 5]**

| Composition of starch paste sample (in 20 g) | | |
|---|---|---|
| | Control diet | Test diet |
| Starch | 2g | 2g |
| Test sample | Control (No addition) | Any one of Test sample Nos. 2 to 4 and 8 (in terms of CA: 175 µM) |
| Triolein | 1 g | 1 g |
| Egg yolk lecithin | 0.04 g | 0.04 g |
| Albumin | 0.2 g | 0.2 g |
| Distilled water | 16.76 g | 16.76 g |

### (Intragastric administration test)

The starch paste sample containing the test sample was intragastrically administered to a mouse fasted for 20 hours, and part of the blood was collected from the orbital venous plexus over time (before the administration, and at 10, 30, 60, and 120 minutes after the administration). The intragastric administration and blood collection were performed under deep anesthesia using isoflurane. The collected blood was subjected to centrifugation, and thereafter the blood plasma was prepared and measured using GIP ELISA kit (EMD Millipore Corporation). The blood GIP amount of each sample after the administration was calculated by measuring the maximum value (Cmax) and the area under the concentration curve (AUC) for 120 minutes. Each analysis result was expressed as a relative value with the control administration group taken as 100, Unpaired Student's t test was used for analysis of each item, and the statistically significant difference was determined to be present when the p value was 0.05 or less.

### (1. Comparison of effectiveness against purified product of cycloartenol)

Starch paste samples respectively containing the control, and the purified product of cycloartenol (Sample No. 2) or the commercially available product 1 (Sample No. 3) were used as the test samples, and the intragastric administration test was conducted. The results are shown in Table 6. A significant reduction action caused by the intragastric administration of the starch paste sample containing the purified product of cycloartenol was recognized in the maximum value (Cmax) of the blood GIP and the area under the concentration curve (AUC) for 120 minutes, as compared with the control group. On the other hand, in the intragastric administration of the starch paste sample containing the commercially available cycloartenol formulation, a significant reduction action on the blood GIP was not recognized.

**[Table 6]**

| Sample | | GIP (Cmax) | GIP (AUC) |
|---|---|---|---|
| - | Control | 100 | 100 |
| 2 | Purified product of cycloartenol | 64 ** | 81 * |
| 3 | Commercially available product 1 | 93 | 84 |

| | | | |
|---|---|---|---|
| *: p<0.05, **: p<0.01 | | | |

### (2. Comparison of effectiveness between samples having different triterpene alcohol composition ratio)

The intragastric administration test was conducted using starch paste samples respectively containing the control and the commercially available products 1 and 2, or the composition A (Sample Nos. 3, 4, and 8) as the test samples. The results are shown in Table 7. No significant blood GIP reduction action was recognized in any one of the test samples having different sterol composition ratio.

**[Table 7]**

| Sample | | GIP (Cmax) | GIP (AUC) |
|---|---|---|---|
| - | Control | 100 | 100 |
| 3 | Commercially available product 1 | 94 | 98 |
| 4 | Commercially available product 2 | 101 | 107 |
| 8 | Composition A (Comparative Example) | 101 | 109 |

From the results of these examples, it has been proved that the purified product of cycloartenol having an inhibitory action against lipase is effective in the blood GIP reduction. Accordingly, the results of these examples show that the cycloartenol-containing compositions of Sample Nos. 6, 7, and 14 to 17 in which the same lipase inhibitory action as in the purified product of cycloartenol was recognized in Example 2 also have the blood GIP reduction action like the purified product of cycloartenol.

## Claims

1. A cycloartenol-containing composition having a cycloartenol content of more than 37% by mass, a 24-methylenecycloartanol content of more than 5% by mass, and a sum content of cycloartenol and 24-methylenecycloartanol of more than 76% by mass, relative to the total mass of sterols.

2. The cycloartenol-containing composition according to claim 1, having a sum content of cycloartenol and 24-methylenecycloartanol of less than 96% by mass, relative to the total mass of sterols.

3. The cycloartenol-containing composition according to claim 1 or 2, having a cycloartenol content of more than 42% by mass, and a sum content of cycloartenol and 24-methylenecycloartanol of more than 81% by mass and less than 96% by mass, relative to the total mass of sterols.

4. The cycloartenol-containing composition according to any one of claims 1 to 3, having both a campesterol content and a sitosterol content of less than 5% by mass, relative to the total mass of sterols.

5. The cycloartenol-containing composition according to any one of claims 1 to 4, the composition being a hydrolysate of alcohol extract of rice bran extract.

6. The cycloartenol-containing composition according to any one of claims 1 to 5, the composition being a composition prepared from the rice bran extract without chromatographic purification.

7. A lipase inhibitor comprising the cycloartenol-containing composition according to any one of claims 1 to 6 as an active ingredient.

8. A lipid absorption inhibitor comprising the cycloartenol-containing composition according to any one of claims 1 to 6 as an active ingredient.

9. A GIP increase inhibitor comprising the cycloartenol-containing composition according to any one of claims 1 to 6 as an active ingredient.

10. An inhibitor of postprandial blood glucose increase comprising the cycloartenol-containing composition according to any one of claims 1 to 6 as an active ingredient.

11. An obesity inhibitor comprising the cycloartenol-containing composition according to any one of claims 1 to 6 as an active ingredient.

12. Use of the cycloartenol-containing composition according to any one of claims 1 to 6 for producing a lipase inhibitor.

13. Use of the cycloartenol-containing composition according to any one of claims 1 to 6 for producing a lipid absorption inhibitor.

14. Use of the cycloartenol-containing composition according to any one of claims 1 to 6 for producing a GIP secretion inhibitor.

15. Use of the cycloartenol-containing composition according to any one of claims 1 to 6 for producing an inhibitor of postprandial blood glucose increase.

16. Use of the cycloartenol-containing composition according to any one of claims 1 to 6 for producing an obesity inhibitor.

17. Non-therapeutic use of the cycloartenol-containing composition according to any one of claims 1 to 6 for lipase inhibition.

18. Non-therapeutic use of the cycloartenol-containing composition according to any one of claims 1 to 6 for lipid absorption inhibition.

19. Non-therapeutic use of the cycloartenol-containing composition according to any one of claims 1 to 6 for GIP secretion inhibition.

20. Non-therapeutic use of the cycloartenol-containing composition according to any one of claims 1 to 6 for inhibition of postprandial blood glucose increase.

21. Non-therapeutic use of the cycloartenol-containing composition according to any one of claims 1 to 6 for obesity inhibition.

22. The cycloartenol-containing composition according to any one of claims 1 to 6 for use in lipase inhibition.

23. The cycloartenol-containing composition according to any one of claims 1 to 6 for use in lipid absorption inhibition.

24. The cycloartenol-containing composition according to any one of claims 1 to 6 for use in GIP secretion inhibition.

25. The cycloartenol-containing composition according to any one of claims 1 to 6 for use in inhibition of postprandial blood glucose increase.

26. The cycloartenol-containing composition according to any one of claims 1 to 6 for use in obesity inhibition.

27. A method for inhibiting lipase, comprising administering the cycloartenol-containing composition according to any one of claims 1 to 6 to a subject in an effective amount.

28. A method for inhibiting lipid absorption, comprising administering the cycloartenol-containing composition according to any one of claims 1 to 6 to a subject in an effective amount.

29. A method for inhibiting GIP secretion, comprising administering the cycloartenol-containing composition according to any one of claims 1 to 6 to a subject in an effective amount.

30. A method for inhibiting postprandial blood glucose increase, comprising administering the cycloartenol-containing composition according to any one of claims 1 to 6 to a subject in an effective amount.

31. A method for inhibiting obesity, comprising administering the cycloartenol-containing composition according to any one of claims 1 to 6 to a subject in an effective amount.
